# EUROPEAN PATENT SPECIFICATION

(11) **EP 1 539 966 B1**
(45) Date of publication and mention of the grant of the patent: **30.06.2010**
(21) Application number: 03750495.8
(22) Date of filing: 08.09.2003
(51) Int. Cl.: C12N 15/82, C12P 21/02, C07K 14/52

(54) **PROTEIN PRODUCTION METHOD**
VERFAHREN ZUR HERSTELLUNG VON PROTEINEN
PROCEDE DE PRODUCTION DE PROTEINES

(30) Priority: 12.09.2002 EP 02020382; 11.07.2003 EP 03015881
(43) Date of publication of application: 15.06.2005
(73) Proprietor: Greenovation Biotech GmbH, 79111 Freiburg (DE)
(72) Inventor: GORR, Gilbert, 79112 Freiburg (DE); LAUNHARDT, Heike, 79106 Freiburg (DE); BERG, Birgit, 79100 Freiburg (DE)
(74) Representative: Stürken, Joachim
(86) International application number: PCT/EP2003/009959
(87) International publication number: WO 2004/024927

(56) References cited:
- WO-A-01/25456
- HOUBA-HERIN NICOLE ET AL: "Cytokinin oxidase from Zea mays: Purification, cDNA cloning and expression in moss protoplasts." PLANT JOURNAL, vol. 17, no. 6, March 1999 (1999-03), pages 615-626, XP002233933 ISSN: 0960-7412
- BHATLA S C ET AL: "Observation of polarity induction by cytochemical localization of phenylalkylamine-binding sites in regenerating protoplasts of the moss Physcomitrella patens." PROTOPLASMA, vol. 219, no. 1-2, 2002, pages 99-105, XP002233934 ISSN: 0033-183X
- GORR G., ET AL.: "Time-to-market for mossbioreactor derived biopharmaceuticals: transient expression in protoplasts for feasibility studies" 6TH INTERLAKEN CONFERENCE ON ADVANCES IN PRODUCTION OF BIOLOGICALS, 25 - 28 March 2003, XP002266913 Interlaken Retrieved from the Internet: <URL:http://www.greenovation.com/pdf/2FA79 16.pdf> [retrieved on 2004-01-14]
- SCHAEFER, D.G.; BISZTRAY, G.; ZRYD, J.P.: "Genetic transformation of the moss Physcomitrella patens." BIOTECHNOLOGY IN AGRICULTURE AND FORESTRY, vol. 29, 1994, pages 349-364, XP008014584 ISSN: 0934-943X
- FIEBICH BERND L ET AL: "Synthesis and assembly of functionally active human vascular endothelial growth factor homodimers in insect cells." EUROPEAN JOURNAL OF BIOCHEMISTRY, vol. 211, no. 1-2, 1993, pages 19-26, XP008014489 ISSN: 0014-2956 cited in the application
- KNIGHT C D ET AL: "MOLECULAR RESPONSES TO ABSCISIC ACID AND STRESS ARE CONSERVED BETWEEN MOSS AND CEREALS" PLANT CELL, AMERICAN SOCIETY OF PLANT PHYSIOLOGISTS, ROCKVILLE, MD, US, vol. 7, no. 5, May 1995 (1995-05), pages 499-506, XP001064674 ISSN: 1040-4651
- SCHAEFER DIDIER G: "A new moss genetics: Targeted mutagenesis in Physcomitrella patens." ANNUAL REVIEW OF PLANT BIOLOGY, vol. 53, 2002, pages 477-501, XP002233936 2002 Annual Reviews 4139 El Camino Way, Palo Alto, CA, 94303-0139, USA ISBN: 0-8243-0653-8
- SCHAEFER D. ET AL.: "Stable transformation of the moss Physcomitrella patens" MOL GEN GENET, vol. 226, 1991, pages 418-424, XP008084955

## Description

### TECHNICAL FIELD

The present invention relates generally to methods and materials for use in gene expression, and in particular for the production of extracellular non-plant protein from moss protoplasts.

### PRIOR ART

Recombinant protein production systems currently in use range from prokaryotic systems such as *Escherichia coli* and *Bacillus*, to eukaryotic systems such as yeast, mammalian cells, insect cell cultures, transgenic animals and plants. Proteins having post-translational modifications, e.g. glycosylation and processing of disulphide bridges, are typically produced in eukaryotic systems. For large-scale production of recombinant proteins typically stably transformed cell lines or organisms (that is, systems wherein the nucleic acid of interest has been integrated into the nuclear genome) have been used, as well as baculovirus/insect cell systems. For example, Schaefer et al. (1991)disclose a method for the stable transformation of *Physcomitrella patens,* comprising culturing the transformed protoplasts in 3M medium for 4 to 7 days prior to regenerating the plant. The establishment of such stably transformed cell lines or organisms is a time consuming and labor intensive process.

Transfection of mammalian cells or insect cells requires many man-hours and the use of specialized technical equipment e.g. CO₂-incubation chamber. Both of these factors make the cost of production very expensive.

Plant-based transient transformation systems that are used for transient expression and production of recombinant proteins are based on the inoculation of plant tissues or whole plants with agrobacteria or with virus particles (Vaquero et al. 1999; Fischer et al. 1999; Fischer and Emans 2000; Dirnberger et al. 2001). Direct DNA transfer into plant tissue by particle bombardment has also been used as a method for testing gene expression prior to stable transformation (Fischer et al. 1999).

Alternative transient transformation systems, that is, systems wherein the nucleic acid of interest is not integrated into the nuclear genome, offer a way forward for the efficient expression of heterologous proteins in commercial quantities.

Up-to now, transient protein expression in protoplasts has only been used with reporter systems for e.g. promoter analysis (Zeidler et al. 1999); analysis of signal transduction; protein targeting and trafficking; and protein-protein interaction (Sheen 2001). Reporter assays have been used in transient transformation experiments with protoplasts for the analysis of signal peptides for protein secretion, (e.g. De Loose et al. 1991, Denecke et al. 1990).

The secretion of recombinant proteins into the extracellular space (apoplast) using stably transformed plants (sporophyte) is well characterized (Magnuson et al. 1996, De Wilde et al. 1998) and prima facie appeared to be a useful tool for protein production in plants. The secretion of recombinant proteins expressed in stably transformed transgenic moss strains (gametophyte) directly into the medium has also been described (WO 01/25456 A2).

Houba-Herin et al. (1999) describe the introduction of a plant (maize) cytokinin oxidase (CKO) into moss protoplasts in a transient assay system and analyzing the role of CKO in plant development. The teaching of the paper describes the expression of a correctly folded and glycosylated plant (maize) cytokinin oxidase and does not suggest the moss protoplast as a possible protein production system.

However, the transient expression and secretion of recombinant non-plant proteins for protein production (sufficient for protein analyses e.g. post-translational modifications, MALDI-TOF analysis) using moss protoplasts transiently transformed via PEG mediated direct DNA transfer does not appear to have been described in the prior art.

Surprisingly, the present inventors have found that transiently transformed moss protoplasts (gametophytes) are able to express high levels of recombinant non-plant proteins which are secreted into the medium. Transiently transformed moss protoplasts can be cultivated in simple media, which may or may not comprise components that are suitable for protein stabilization. Furthermore, the moss protoplasts can be concentrated to a high cell density which enables them to be transiently transformed relatively easily and in high numbers for use in systems wherein low volumes of culturing medium may be employed allowing the production of concentrated protein solutions facilitating further analyses which require small volumes but high protein concentrations (e.g. post-translational modifications).

### DISCLOSURE OF THE INVENTION

According to the present invention there is provided a method for the transient production of (an) extracellular non-plant protein(s) in a moss protoplast, which method comprises the steps of transiently transforming the protoplast with a nucleic acid construct comprising (a) heterologous nucleotide sequence(s) coding for said non-plant protein(s) operably linked to a promoter, and cultivating the transformed protoplast in order to produce said non-plant protein(s), wherein said cultivation takes place for at least 48 hours in a culture medium selected from the group consisting of W5, 3M and MMS.

Particular aspects of the invention will now be discussed in more detail.

### Definitions

The term "heterologous" is used broadly below to indicate that the gene/sequence of nucleotides in question have been introduced transiently into moss protoplasts using genetic engineering, i.e. by human intervention. A heterologous sequence of nucleotides may comprise the coding sequence for a fusion protein comprised of a fusion partner that may be formed, for example, in part by a plant protein that may be fused to a non-plant protein which may be termed a hybrid plant: non-plant fusion protein for the purposes of the present invention. Such a hybrid fusion protein may also be considered as a non-plant protein for the purposes of the present invention. Alternatively, a fusion protein may be one which is formed of fusion partners that are of non-plant origin. A heterologous gene may augment the expression of a protein of interest from an endogenous equivalent gene, i.e. one which normally performs the same or a similar function, or the inserted sequence may be additional to the endogenous gene or other sequence. Nucleic acid heterologous to a cell may be non-naturally occurring in moss protoplasts of that type, variety or species. Thus the heterologous nucleic acid may comprise a coding sequence of, or derived from, a particular type of organism, such as a mammalian species, e.g. of human, ovine, bovine, equine, or porcine species, placed within the context of a moss protoplast, such as a protoplast derived from *Physcomitrella patens.* A further possibility is for a nucleic acid sequence to be placed within a moss protoplast in which it or a homologue is found naturally, but wherein the nucleic acid sequence is linked and/or adjacent to nucleic acid which does not occur naturally within the cell, or cells of that type or species or variety of plant, such as operably linked to one or more regulatory sequences, such as a promoter sequence, for control of expression.

"Gene" unless context demands otherwise refers to any non-plant nucleic acid encoding genetic information for translation into a peptide, polypeptide or protein.

"Vector" is defined to include, inter alia, any plasmid, cosmid, phage, or viral vector in double or single stranded linear or circular form which may or may not be self transmissible or mobilizable, and which can transform a prokaryotic or eukaryotic host and exists extrachromosomally (e.g. autonomous replicating plasmid with an origin of replication). Specifically included are shuttle vectors by which is meant a DNA vehicle capable, naturally or by design, of replication in two different host organisms, which may be selected from actinomycetes and related species, bacteria and eucaryotic (e.g. higher plant, mosses, mammalian, yeast or fungal) cells.

"Expression vector" refers to a vector in which a nucleic acid is under the control of, and operably linked to, an appropriate promoter or other regulatory elements for transcription in a host cell such as a microbial cell or a moss protoplast. The vector may be a bifunctional expression vector which functions in multiple hosts. In the case of genomic or subgenomic DNA, this may contain its own promoter or other regulatory elements and in the case of cDNA this may be under the control of an appropriate promoter or other regulatory elements for expression in the host cell.

A "promoter" is a sequence of nucleotides from which transcription may be initiated of DNA operably linked downstream (i.e. in the 3' direction on the sense strand of double-stranded DNA).

"Operably linked" means joined as part of the same nucleic acid molecule, suitably positioned and oriented for transcription to be initiated from the promoter.

The term "inducible" as applied to a promoter is well understood by those skilled in the art. In essence, expression under the control of an inducible promoter is "switched on" or increased in response to an applied stimulus. The nature of the stimulus varies between promoters. Some inducible promoters cause little or undetectable levels of expression (or no expression) in the absence of the appropriate stimulus. Other inducible promoters cause detectable constitutive expression in the absence of the stimulus. Whatever the level of expression is in the absence of the stimulus, expression from any inducible promoter is increased in the presence of the correct stimulus.

The invention also embraces use of a variant of any of these sequences. A variant protein shares homology with, or is identical to, all or part of the sequences discussed above. Generally speaking, wherever the term is used herein, variants may be:
(i) naturally occurring homologous variants of the relevant non-plant protein,
(ii) artificially generated homologous variants (derivatives) which can be prepared by the skilled person in the light of the present disclosure, for instance by site directed or random mutagenesis, or by direct synthesis. Preferably the variant nucleic acid, encoding the variant polypeptide, is generated either directly or indirectly (e.g. via one or more amplification or replication steps) from an original nucleic acid. Changes to the nucleic acid sequence may produce a derivative by way of one or more of addition, insertion, deletion or substitution of one or more nucleotides in the nucleic acid, leading to the addition, insertion, deletion or substitution of one or more amino acids in the encoded polypeptide. Desirable mutation may be random or site directed mutagenesis in order to alter the activity (e.g. specificity) or stability of the encoded polypeptide. Changes may be by way of conservative variation, i.e. substitution of one hydrophobic residue such as isoleucine, valine, leucine or methionine for another, or the substitution of one polar residue for another, such as arginine for lysine, glutamic for aspartic acid, or glutamine for asparagine. Also included are variants having non-conservative substitutions. In regions which are critical in determining the peptides conformation or activity such changes may confer advantageous properties on the polypeptide e.g. altered stability or specificity.

Similarity or homology in the case of variants is preferably established via sequence comparisons made using FASTA and FASTP (see Pearson & Lipman, 1988. Methods in Enzymology 183: 63-98). Parameters are preferably set, using the default matrix, as follows:
Gapopen (penalty for the first residue in a gap): -12 for proteins / -16 for DNA
Gapext (penalty for additional residues in a gap): -2 for proteins / -4 for DNA
KTUP word length: 2 for proteins / 6 for DNA.

Homology may be at the nucleotide sequence and/or encoded amino acid sequence level. Preferably, the nucleic acid and/or amino acid sequence shares at least about 75%, or 80% identity, most preferably at least about 90%, 95%, 96%, 97%, 98% or 99% identity.

Homology may also be assessed by use of a probing methodology (Sambrook et al., 1989). One common formula for calculating the stringency conditions required to achieve hybridization between nucleic acid molecules of a specified sequence homology is: Tₘ = 81.5°C + 16.6Log [Na+] + 0.41 (% G+C) - 0.63 (% formamide) - 600/#bp in duplex. As an illustration of the above formula, using [Na+] = [0.368] and 50-% formamide, with GC content of 42% and an average probe size of 200 bases, the Tₘ is 57°C. The Tₘ of a DNA duplex decreases by 1 - 1.5°C with every 1% decrease in homology. Thus, targets with greater than about 75% sequence identity would be observed using a hybridization temperature of 42°C.

### Use in mosses

As described below, in its various aspects, the invention will generally be employed on moss protoplasts, using nucleic acids encoding non-plant proteins of interest.

Suitable promoters which operate in moss protoplasts include the Cauliflower Mosaic Virus 35S (CaMV 35S). Other examples are disclosed at pg 120 of Lindsey & Jones (1989) Plant Biotechnology in Agriculture@ Pub. OU Press, Milton Keynes, UK. The promoter may be selected to include one or more sequence motifs or elements conferring developmental and/or tissue-specific regulatory control of expression. Inducible plant promoters include the ethanol induced promoter of Caddick et al (1998) Nature Biotechnology 16: 177-180.

If desired, selectable genetic markers may be included in the construct, such as those that confer selectable phenotypes such as resistance to antibiotics or herbicides (e.g. kanamycin, hygromycin, phosphinotricin, chlorsulfuron, methotrexate, gentamycin, spectinomycin, imidazolinones and glyphosate).

The present invention also provides methods comprising the transient introduction of such constructs comprising appropriate heterologous sequences into a moss plant cell and/or induction of expression of a construct within a moss plant cell, by application of a suitable stimulus e.g. an effective exogenous inducer. Suitable moss plant cells include the moss protoplast, such as those derived from *Physcomitrella patens.*

Nucleic acid can be introduced into moss protoplasts using any suitable technology, such as PEG-mediated DNA uptake as herein described, particle or microprojectile bombardment (US 5100792, EP-A-444882, EP-A-434616) microinjection (WO 92/09696, WO 94/00583, EP 331083, EP 175966, Green et al. (1987) Plant Tissue and Cell Culture, Academic Press), electroporation (EP 290395, WO 8706614 Gelvin Debeyser) other forms of direct DNA uptake (DE 4005152, WO 9012096, US 4684611), liposome mediated DNA uptake (e.g. Freeman et al. Plant Cell Physiol. 29: 1353 (1984)), or the vortexing method (e.g. Kindle, PNAS U.S.A. 87: 1228 (1990d) Physical methods for the transformation of plant cells are reviewed in Oard, 1991, Biotech. Adv. 9: 1-11.

Electroporation, PEG-mediated DNA uptake and direct DNA uptake are preferred. Especially preferred is the modified PEG mediated DNA uptake procedure as disclosed in the examples herein.

The particular choice of a transformation technology will be determined by its efficiency to transform certain moss species as well as the experience and preference of the person practising the invention with a particular methodology of choice. It will be apparent to the skilled person that the particular choice of a transient transformation system to introduce nucleic acid into moss protoplasts is not essential to the invention. However, the use of the PEG-mediated DNA transformation system as described herein is preferred.

Thus various aspects of the present invention provide a method of transiently transforming a moss protoplast involving introduction of a heterologous nucleic acid-based construct as described below into a moss protoplast and causing or allowing transient expression from the vector that results in turn to the secretion of extracellular protein into the medium. Cultivation of protoplasts in a highly concentrated manner results in efficiently improved protein concentrations.

In a preferment there is provided a method of transiently transforming a moss protoplast by PEG mediated DNA uptake and cultivation thereof **characterised in that** the cultivation is conducted in a culture volume of up to 1000µl. Preferably, the cultivation is carried out in a cultivation volume of from 50 - 1000µl, more preferably from 100 - 600µl, more preferably still from 100 - 300µl and most preferably in about 200µl +/- 50µl.

### Choice of genes to enhance

Genes of interest include those encoding non-plant proteins which are themselves, natural medicaments such as pharmaceuticals or veterinary products.

Heterologous nucleic acids may encode, *inter alia,* genes of bacterial, fungal, or non-plant origin such as fusion proteins as alluded to hereinabove or animal origin. Polypeptides produced may be utilised for producing polypeptides which can be purified therefrom for use elsewhere. Proteins that can be produced in a process of the invention include heterodimers, such as FSH, immunoglobulins, fusion antibodies and single chain antibodies.

Such proteins include, but are not limited to retinoblastoma protein, p53, angiostatin, and leptin. Likewise, the methods of the invention can be used to produce mammalian regulatory proteins. Other sequences of interest include proteins, hormones, such as follicle stimulating hormone, growth factors, cytokines, serum albumin, haemoglobin, collagen, thaumatin, thaumatin-like proteins, epidermal growth factors such as VEGF, etc.

### Expression of target genes

Generally speaking, heterologous nucleic acids may be expressed by any appropriate process used in the art or they may be transcribed or expressed as follows:
(i) transient expression of 'naked' DNA e.g. comprising a promoter operably linked to the heterologous sequence of interest,
(ii) expression from an expression vector, such as a replicating vector. Generally speaking, those skilled in the art are well able to construct vectors and design protocols for transient recombinant gene expression. Suitable vectors can be chosen or constructed, containing appropriate regulatory sequences, including promoter sequences, terminator fragments, polyadenylation sequences, enhancer sequences, marker genes and other sequences as appropriate. For further details see, for example, Molecular Cloning: a Laboratory Manual: 2nd edition, Sambrook et al, 1989, Cold Spring Harbor Laboratory Press or Current Protocols in Molecular Biology, Second Edition, Ausubel et al. eds., John Wiley & Sons, 1992.
(iii) expression from a non-integrating vector which is at least transiently present in the cytoplasm,

It will be understood that these categories are not mutually exclusive, for instance because a non-integrating vector may also be an expression vector etc.

Methods for achieving such expression are discussed elsewhere herein.

### ENHANCED TRANSIENT EXPRESSION FROM NON-INTEGRATED VECTORS

As discussed above, the present inventors have discovered that enhanced expression from constructs introduced (preferably at high levels) into the protoplasts of a moss, preferably at high cell density, such as *Physcomitrella patens,* which constructs are non-integrated into the genome (e.g. ectopically) give rise to transcribed mRNA. Constructs can be introduced at relatively high copy number with strong promoters, and without the inherent moderating effect which may occur when selecting a stable transformant in which a construct is integrated into the genome. As a result the levels and concentrations of protein produced may far exceed those obtainable by use of methods for protein production in plant cell based systems of the prior art.

Thus in one aspect of the invention there is disclosed use of a transiently transformed moss protoplast capable of generating mRNA encoding a target protein generated by transcription from a transiently introduced nucleic acid construct including the target nucleotide sequence operably linked to a promoter, which construct is introduced into the cell of an organism.

The "introduced nucleic acid" will thus include the heterologous nucleic acid sequence as a DNA sequence provided in the form of a construct that is capable of giving rise to the production of extracellular protein at an elevated level relative to the level of protein production normally associated with stable transgene expression of the said DNA sequence. In one aspect of the invention, the heterologous nucleic acid sequence may encode a protein that is made up of a signal and/or a transit peptide coupled to the protein or polypeptide sequence of choice.

Thus in a preferred aspect of the invention, there is disclosed a method of achieving transient expression of an heterologous nucleotide sequence in a plant, which method comprises the step of transiently introducing into a moss protoplast at least a first nucleic acid sequence comprising a heterologous non-plant nucleotide sequence.

In one embodiment there is provided a method of generating at least an extracellular heterologous non-plant protein, which method comprises the steps of:
(i) transiently introducing into a protoplast of a moss a first nucleic acid comprising the nucleotide sequence coding for the heterologous protein,
(ii) causing or permitting expression from the nucleic acid, over a period of time, of the heterologous protein,
(iii) harvesting the secreted heterologous protein from the medium,
(iv) optionally isolating the moss protoplasts from the medium.

As a result, the moss protoplasts may be used to continuously produce desired proteins after each harvesting of the secreted protein.

The isolation may be by entirely conventional means, and may or may not entail partial or complete purification.

The moss protoplasts or tissues will generally comprise gametophyte cells, such as gametophytes of the moss, *Physcomitrella patens.*

The time period for the moss protoplast culture may be any period up to or even beyond which the tissue remains viable, or until it is saturated with protein; in general it may be preferred that it is between about 1 to 10 days, more preferably between about 1 to 5 days.

Naturally, the man skilled in the art will recognise that more than one gene may be used in the, or each, construct, although a single sequence in each case is preferred. Multiple vectors (each including one or more nucleotide sequences encoding heterologous protein of choice) may be introduced into the moss protoplasts via PEG-mediated DNA uptake methods as described herein. This may be useful for producing e.g. multiple subunits e.g. of an enzyme.

As shown in the Examples below, transient expression of the heterologous sequence when introduced in this way can give very high levels of target polypeptide over the course of the transient expression period, which will generally be several days, depending on the precise methods and materials employed. By using the methods of the invention as herein described, heterologous polypeptide secretion into the medium is achieved.

Thus transient expression in the moss protoplast represents a useful tool in many contexts for which it may previously have been considered unsuitable e.g. dependable expression of unstable, that is, transiently expressed heterologous protein or polypeptide sequences that are secreted into the medium.

The method may be particularly preferred in those applications where high levels of expression are required, but where viral constructs (with the requirement for plant 'infection') or stable transgenic plants are undesirable e.g. where a rapid assay is important, or where the sequence in question imparts a lethal phenotype.

The reporter can be any detectable protein, such as a marker gene, commonly used in the art such as GUS, GFP, luciferase etc. Preferably, the reporter is a non-invasive marker such as GFP or luciferase.

The invention will now be further described with reference to the following non-limiting Figures and Examples. Other embodiments of the invention will occur to those skilled in the art in the light of these.

### EXAMPLES

### Methods and Materials

### Plant material

The wild-type strain of *Physcomitrella patens* (Hedw.) B.S.G. characterised previously by Reski et al. 1994 was used. It is a subculture of strain 16/14 which was collected by H.L.K. Whitehouse in Gransden Wood, Huntingdonshire, UK and was propagated by Engel (1968).

### Construction of vectors

### Construction of pRT101 To32

*PCR-screening for derived polynucleotide sequences. A Thuja occidentalis* cDNA-library was screened with degenerate primers for specific clones by PCR. To do so, 5'-primers were designed according to the N-terminal sequence obtained by Edman degradation (Edman and Begg G, 1967) of the purified endogenous To32 protein, the 3'-primers were derived from the consensus motif of thaumatin-like proteins: 50 ng of *Thuja occidentalis* cDNA-library were used as a template in a Pfu-proof-reading-PCR (Promega, Germany; PCR conditions: 5 min 94°C; 30 cycles: 30s 94°C/45s 50°C/90 s 72°C; extension: 10 min) using the 5'-primer To32N-2-S (5'-TTYGAYATIMGIAAYCARCAYGGNTAYAC-3')and the 3'-primer Thaum-Mot-2-AS (5'-TCYTKIGSRTAGCTRTAVGCITSDGGRCA-3'). The amplified PCR-product was purified according to standard procedures (see Sambrook and Russell, 2001). 1 µl of 1/100-diluted PCR-product was reamplified in a "nested" Pfu-proof-reading-PCR (Promega, Germany; PCR conditions: 5 min 94°C; 30 cycles: 30s 94°C/45s 50°C/90 s 72°C; extension: 10 min) with 5'-primer To32-N-3-S (5'-GCNGCIGGNYTNCCNGGIGGIGGNCA-3') and 3'-primer Thaum-Mot-1-AS (5'-CCRTCRAYIAIMGAIAYGTCSYAGAAATC-3'). PCR products were analyzed by 2% (w/v) agarose gel electrophoresis and a 263 bp-band was excised and purified from the gel (Quiagen, Hilden, Germany). It was cloned into vector pCR4-TOPO according to the manufacturer's protocol (Invitrogen, Carlsbad, CA, USA; protocol book: versionH, 072701; 25-0276) for sequencing. In detail: To 4 µl of purified, adenylated (by Taq-PCR-extension; see protocol) PCR-product 1 µl of salt solution and 1 µl of TOPO vector was added. The gently mixed reaction was incubated for 5 min at room temperature and then transformed into chemo-competent Top10 cells (for method see Sambrook and Russell, 2001). Positive clones were selected by plating on LB-plates, containing 50 µg/ml kanamycin. The resulting vector pCRTo was sequenced and contained a 263 bp-fragment (bp121 - 384 in CDS) of the To32-cDNA. From this sequence a new 5'-primer To32-300-1-S (5'-GGCAGGGACAAAGAAGGC-3') and a new 3'-primer To32-300-2-AS (5'-AGTGTGCAGCTCAGTTGGC-3') were derived.

To obtain the missing 5'-sequence of To32 a *Pfu*-proof-reading-PCR (Promega, Germany; PCR conditions: 5 min 94°C; 30 cycles: 30s 94°C/45s 50°C/90 s 72°C; extension: 10 min) was performed with 50 ng *Thuja occidentalis* cDNA-library, the 5'-primer T7term (5'-CTAGTTATTGCTCAGCGG-3') and the 3'-primer To32-300-2-AS (see above). The purified PCR-product was cloned into PCR4-TOPO according to the manufacturer's protocol (Invitrogen, Carlsbad, CA, USA; protocol book: versionH, 072701; 25-0276), resulting in vector pCRToN, and sequenced. This vector contains the first 295 bp of the To32-CDS (bp1-295). To obtain the missing 3'-sequence of To32 a Pfu-proof-reading-PCR (Promega, Germany; PCR conditions: 5 min 94°C; 30 cycles: 30s 94°C/45s 50°C/90 s 72°C; extension: 10 min) was performed with 50 ng *Thuja occidentalis* cDNA-library, the 5'-primer To32-300-1-S (see above) and the 3'-primer pYES-2-AS (5'-AACTAATTACATGATGCGGC-3'). The purified PCR-product was cloned into PCR4-TOPO according to the manufacturer's protocol (Invitrogen, Carlsbad, CA, USA; protocol book: versionH, 072701; 25-0276), resulting in vector pCRToC, and sequenced. This vector contains the 3'-region of the To32-CDS (bp185-693). CDNA-sequences for To32 were overlapping in vectors pCRTo, pCRToN and PCRToC. Thus, the whole CDS for To32 could be identified, comprising 693 bp. This could be verified by sequencing of pETTo32 (see cloning procedures for To32 in pET24b(+) below), which was 100 % identical. In this case the CDS, coding for the native protein was again amplified from the *Thuja occidentalis* cDNA library.

NB for the degenerate primers described above: R = G or A; Y = C or T; W = A or T; M = A or C; K = G or T; I = inosine; S = C or G; N = G,A,T or C.

*Cloning procedures for To32 in pET24 b*(+) (Novagen, Madison,WI, USA). The 615 bp-coding sequence of To32, non-comprising the sequence coding for the 26 amino acid leader peptide,was amplified from the *Thuja occidentalis* library by *Pfu*-proof-reading PCR (Promega, Germany) using the upstream primer To32Nterm-BamH1-S (5'-CGG GAT CCA GTG AAG TTT AAT ATA CGG AAC C-3') and the downstream primer To32Cterm-EcoRI1-AS (5'-CGG AAT TCG GGC AGA ATA CGA TAC TGT AGT C-3'). After restriction of the resulting amplification product with *Eco*RI and *Bam*H1 it was cloned into prokaryotic expression vector *pET24b(+),* resulting in *pETTo32.*

*Cloning procedures for To32 in pGEX-4T-1.* The 615 bp-cDNA-sequence was amplified from *pETTo32.* An *Eco*RI/*Xho*I-restricted PCR-product was cloned into *pGEX-4T-1,* using the primers P33 (5'-ATCGAATTCGTGAAGTTTAATATACGG-3') and P34 (5'-GTCCTCGAGTTA GGGGCAGAATACGATAC-3') resulting in *pGEXTo32.*

*Cloning procedures for To32 in pKSTo32.* A 150 bp-cDNA-sequence coding for To32 N-terminus (including the 78 bp sequence coding for To32 signal peptide) was amplified from pCRToN using the primers P72 (5'-GTC GAA TTC ATG GCC ACA GTT TCA GAT C -3') and P74 (5'- AAG CAG CTG GAC CAG GGT CAG AC -3'). The *Eco*RI/*Pvu*II restricted PCR-product was cloned in *pKs* (*Eco*RI / *Not*I digested), in a one tube ligation including a *Pvu*II / *Not*I digested 546 bp-fragment derived from *pGEXTo32,* resulting in *pKSTo32. pKSTo32* contained the cDNA-sequence of thaumatin-like protein To32 including the coding sequence of the signal peptide.

*Cloning procedures for To32 in pRT101To32.* To32 cDNA was amplified from the plasmid pKsTo32 using the primers MoB254 (5'- ATA CTC GAG GAA GAT GGC CAC AGT TTC -3') and P73 (5'- GTC GGA TCC TTA CTT GTC GTC GTC ATC CTT GTA GTC GGG GCA GAA TAC GAT ACT G -3') by Pfu-proof-reading-PCR (Promega, Germany; PCR conditions: 2 min 94°C; 25 cycles: 30 s 94°C/30 s 54°C/2 min 72°C; extension: 10 min). After restriction of the resulting amplification product with *Xho*I and *Bam*HI it was cloned into pRT101 (Töpfer et al. 1987; *Xho*I / *Bam*HI digested). The resulting plasmid contained the To32 cDNA (including coding sequences of To32 signal peptide and C-terminal FLAG epitiope) positioned downstream of CaMV 35 S promoter and terminated by CaMV terminator. This plasmid was named pRT101To32.

### Construction of pRT101VEGF C3

Human vascular endothelial growth factor 121 (VEGF₁₂₁) cDNA without leader sequence was excised as an *Nde*I-*Sal*I fragment from pCYTEXP-VEGF₁₂₁ (kindly provided from Dr McCarthy, GBF, Braunschweig, Germany). This fragment was blunted by the Klenow reaction and introduced into pRT101 (Töpfer et al. 1987) at the *Sma*I restriction site to form plasmid pRT101VEGF C3. In this construct, the VEGF₁₂₁ cDNA without leader sequence was placed downstream of the CaMV 35 S promoter and terminated by the CaMV terminator (Gorr, 1999).

### Construction of pRT101TPVEGF C3

The sequence for VEGF signal peptide (sorting signal for secretion) was cloned into pRT101VEGF C3. The signal peptide cDNA was amplified from the plasmid pRT101 P21 (Gorr, 1999) using the 5' primer MoB323 5'- ATA CTC GAG GAA GAT GAA CTT TTC TGC CTG TCT TGG -3' containing an *Xho*I restriction side and 3' primer MoB349 5'- CTG CCA TGG GTG CAG CCT GGG ACC AC -3' containing *Nco*I restriction side. The amplified DNA was digested with *Xho*I and *Nco*I and ligated into pRT101VEGF C3 (*Xho*I / *Nco*I digested) resulting in pRT101TPVEGF C3. The resulting plasmid contained the coding sequences for the VEGF signal peptide and VEGF₁₂₁ in frame under control of CaMV 35 S promoter.

### Construction of pRT101TPVEGF-1 C3

The sequence for VEGF signal peptide (sorting signal for secretion) without 3'-terminal three nucleotides (causing a deletion of the last amino acid (C-terminus) of the transit peptide) was cloned into pRT101VEGF C3. The signal peptide cDNA was amplified from the plasmid pRT101 P21 (Gorr, 1999) using the 5' primer MoB323 5'- ATA CTC GAG GAA GAT GAA CTT TTC TGC CTG TCT TGG -3' containing an *Xho*I restriction side and 3' primer MoB350 5'- CTG CCA TGG GTG CAG CCT GGG ACC ACT TGG -3' containing *Nco*I restriction side and 3' deletion. The amplified DNA was digested with *Xho*I and *Nco*I and ligated into pRT101VEGF C3 (*Xho*I / *Nco*I digested) resulting in pRT101TPVEGF-1 C3. The resulting plasmid contained the coding sequences for the VEGF signal peptide including the 3' deletion and VEGF₁₂₁ in frame under control of CaMV 35 S promoter.

### Construction of pRT101TPTo32 C3

The sequence for thaumatin-like protein To32 signal peptide (sorting signal for secretion) was cloned into pRT101VEGF C3. The signal peptide cDNA was amplified from the plasmid pCRToN (pTo63) using the 5' primer MoB254 5'- ATA CTC GAG GAA GAT GGC CAC AGT TTC AGA TC - 3' containing an *Xho*I restriction side and 3' primer MoB250 5'- CTG CCA TGG GTG CTG CTC CCG CCT CT -3' containing *Nco*I restriction side. The amplified DNA was digested with *Xho*I and *Nco*I and ligated into pRT101VEGF C3 (*Xho*I / *Nco*I digested) resulting in pRT101TPTo32 C3. The resulting plasmid contained the coding sequences of To32 signal peptide and VEGF₁₂₁ in frame under control of CaMV 35 S promoter.

### Construction of pRT 101TPTo32-1 C3

The sequence for thaumatin-like protein To32 signal peptide (sorting signal for secretion) without 3'-terminal three nucleotides (causing a deletion of the last amino acid (C-terminus) of the transit peptide) was cloned into pRT101VEGF C3. The signal peptide cDNA was amplified from the plasmid pCRToN (pTo63) using the 5' primer MoB254 5'- ATA CTC GAG GAA GAT GGC CAC AGT TTC AGA TC -3' containing an *Xho*I restriction side and 3' primer MoB324 5'- CTG CCA TGG GTG CTC CCG CCT CTT GG -3' containing *Nco*I restriction side. The amplified DNA was digested with *Xho*I and *Nco*I and ligated into pRT101VEGF C3 (*Xho*I / *Nco*I digested) resulting in pRT101TPTo32-1 C3. The resulting plasmid contained the coding sequences of for the To32 signal peptide including 3' deletion and VEGF₁₂₁ in frame under control of CaMV 35 S promoter.

### Construction of pRT99TPftszGFP

Expression cassette for GFP containing 297-bp *fts*Z1 cDNA fragment coding for the FtsZ1 chloroplast transit peptide and cDNA for *gfp* under control of CaMV 35 S promoter and nos terminator was excised as a *Hind*III / *Eco*RI fragment from pFtsZ1(1-93)-GFP (Kiessling et al. 2000). The fragment was ligated into pRT99 ( *Hind*III / *Eco*RI digested) resulting in pRT99TPftszGFP.

### Standard culture conditions

Plants were grown axenicallly under sterile conditions in plain inorganic liquid modified Knop medium (1000 mg/l Ca(NO₃)₂ x 4H₂O 250 mg/l KCl, 250 mg/l KH₂PO4, 250 mg/l MgSO₄ x 7 H₂O and 12.5 mg/l FeSO₄ X 7 H₂O; pH 5.8 (Reski and Abel 1985)). Plants were grown in 500 ml Erlenmeyer flasks containing 200 ml of culture medium and flasks were shaken on a Certomat R shaker (B.Braun Biotech International, Germany) set at 120 rpm. Conditions in the growth chamber were 25 +/- 3°C and a light-dark regime of 16:8 h. The flasks were illuminated from above by two fluorescent tubes (Osram L 58 W / 25) providing 35 µmols⁻¹m⁻². The cultures were subcultured once a week by disintegration using an Ultra-Turrax homogenizer (IKA, Staufen, Germany) and inoculation of two new 500 ml Erlenmeyer flasks containing 100 ml fresh Knop medium.

### Protoplast isolation

Preculture of moss tissue for optimal protoplast isolation. Mosses (especially *Physcomitrella patens*) can be precultured under different conditions to obtain optimal protoplast yields:
I. Rother et al. 1994 cultivated moss tissue for 7 days in Knop medium with reduced (10%) Ca(NO₃)₂ content. Cultures were filtered 3 or 4 days after disintegration and were transferred into fresh Knop medium with reduced (10%) Ca(NO₃)₂ content.
II. Instead of reduction of Ca(NO₃)₂ the medium for preculture can be supplemented with 5 mM ammonium tartrate or the pH can be shifted to 4.5 (in liquid cultures with uncontrolled pH-values an average pH of 5.8 is reached for modified Knop medium). Cultures were filtered 3 or 4 days after disaggregation of tissue and were transferred into fresh modified Knop medium (supplemented with 5 mM ammonium tartrate or shifted to pH 4.5).
III. Hohe and Reski (2002) optimised culture conditions in a semicontinous bioreactor to obtain high yields of protoplasts. Isolated protoplasts of high yields were obtained either by supplementation of modified Knop medium (Reski and Abel 1985) with 460 mg/l ammonium tartrate or under controlled pH-values with a setpoint of 4.5 (in bioreactor cultures with uncontrolled pH-values an average pH of 5.8 is reached for modified Knop medium).

Different protocols for the isolation of protoplasts (Grimsley et al. 1977; Schaefer et al. 1991; Rother et al. 1994; Zeidler et al. 1999; Hohe and Reski 2002, Protocol Schaefer 2001) and for transformation (Schaefer et al. 1991; Reutter and Reski 1996, Protocol Schaefer 2001) have been described for *Physcomitrella patens*.

For the work presented herein, a modification/combination of the previously described methods was used:
After filtration the moss protonemata were preincubated in 0.5 M mannitol. After 30 min, 4 % Driselase (Sigma, Deisenhofen, Germany) was added to the suspension. Driselase was dissolved in 0.5 M mannitol (pH 5.6-5.8), centrifuged at 3600 rpm for 10 min and sterilised by passage through a 0.22 µm filter (Millex GP, Millipore Corporation, USA). The suspension, containing 1% Driselase (final concentration), was incubated in the dark at RT and agitated gently (best yields of protoplasts were achieved after 2 hours of incubation) (Protocol Schaefer 2001). The suspension was passed through sieves (Wilson, CLF, Germany) with pore sizes of 100 µm and 50 µm. The suspension was centrifuged in sterile centrifuge tubes and protoplasts were sedimented at RT for 10 min at 55 g (acceleration of 3; slow down at 3; Multifuge 3 S-R, Kendro, Germany) (Protocol Schaefer 2001). Protoplasts were gently resuspended in W5 medium (125 mM CaCl₂ x 2H₂O; 137 mM NaCl; 5.5 mM glucose; 10 mM KCl; pH 5.6; 660-680 mOsm; sterile filtered). The suspension was centrifuged again at RT for 10 min at 55 g (acceleration of 3; slow down at 3; Multifuge 3 S-R, Kendro, Germany) (modification, remark: Schaefer et al. 1991 mentioned that W5 medium resulted in reduced viability). Protoplasts were gently re-suspended in W5 medium (Rother et al. 1994). For counting protoplasts a small volume of the suspension was transferred to a Fuchs-Rosenthal-chamber.

### Transformation protocol

For transformation protoplasts were incubated on ice in the dark for 30 minutes. Subsequently, protoplasts were sedimented by centrifugation at RT for 10 min at 55 g (acceleration of 3; slow down at 3; Multifuge 3 S-R, Kendro). Protoplasts were resuspended in 3M medium (15 mM CaCl₂ x 2H₂O; 0.1 % MES; 0.48 M mannitol; pH 5.6; 540 mOsm; sterile filtered, Schaefer et al. 1991) at a concentration of 1.2 x 10⁶ protoplasts / ml (Reutter and Reski 1996). 250 µl of this protoplast suspension were dispensed into a new sterile centrifuge tube, 50 µl DNA solution (column purified DNA in H₂O (Qiagen, Hilden, Germany); 10-100 µl; optimal DNA amount of 60 µg) was added and finally 250 µl PEG-solution (40% PEG 4000; 0.4 M mannitol; 0.1 M Ca(NO₃)₂; pH 6 after autoclaving) was added. The suspension was immediately but gently mixed and then incubated for 6 min at RT with occasional gentle mixing. The suspension was diluted progressively by adding 1, 2, 3 and 4 ml of 3M medium. The suspension was centrifuged at 20°C for 10 minutes at 55 g (acceleration of 3; slow down at 3; Multifuge 3 S-R, Kendro). The pellet was re-suspended in 300 µl or 400 µl medium (for examples: 3M medium or W5 medium or MMS (containing 610 mg MgCl₂ x 6H₂O; 0.2 g MES; 18.21 g D sorbit (Roth, Germany), pH 5.6-5.8; 591 mOsm) medium. Cultivation of transformed protoplasts was performed in 96 well plates (300 µl, Nunclon, Nunc GmbH, Wiesbaden , Germany) or 48 well plates (400 µl, Cellstar, greiner bio-one, Frickenhausen, Germany).

Transient transformation samples were incubated in dim light (4.6 µmols⁻¹m⁻²) at 25°C.

### Sampling:

Medium was carefully replaced (to avoid dispersing protoplasts) by fresh medium every 24 h. The medium was not replaced completely since the protoplasts have to be kept in liquid. The removed medium (including recombinant protein) was stored at -20°C.
Time course experiments were performed by replacing half the medium (200 µl were removed, 200 µl fresh medium were added).
In some experiments protoplasts as well as medium were isolated after the second 24 h.
Samples were stored at -20°C.

### Assays:

### Quantification of recombinant VEGF₁₂₁

Recombinant VEGF₁₂₁ expressed by transient transformed moss protoplasts was quantified by ELISA (R&D Systems, Wiesbaden, Germany). The ELISA was performed according to the instructions of the manufacturer. The samples were diluted for quantification.

Determination of biological activity of recombinant VEGF₁₂₁ Biological activity of recombinant VEGF₁₂₁ expressed by transiently transformed moss protoplasts was determined by VEGF specific RELIDA (RELIATech, Braunschweig, Germany). The RELIDA was performed according to the manufacturer's instructions. The assay recognizes only free, uncomplexed and biologically active forms of VEGF-A that are not sequestered by soluble receptors. Sequestered, monomeric and degraded VEGF-A molecules will not be detected (manufacturer's instructions).

### Western blot analysis

Protein expression was analysed by Western blot analyses. Samples were mixed with reducing or nonreducing sample buffer and resolved on a 12% or a 15% SDS-polyacrylamide gel according to Laemmli (1970). For Western Blotting proteins were electrotransferred from SDS-PAGE by semi dry tranfer to Optitran BA-S83 Reinforced NC Membrane (Schleicher & Schuell, Dassel, Germany) according to Towbin (1979) using a Multiphor II device (Amersham Pharmacia Biotech, Germany). The analysis was carried out using the ECL-detection system (Amersham Pharmacia Biotech, Germany), according to the manufacturer's instructions.

VEGF₁₂₁ detection was performed under non-reducing conditions. Samples were heated for 10 min at 60°C before loading. Recombinant VEGF was detected by using anti rhVEGF antibody (AF-293-NA, R&D Systems, Wiesbaden, Germany) diluted 1/500 in Tris-buffered Saline, pH 8/0.05% Tween 20 and an anti-goat IgG, peroxidase labeled (A5420, Sigma, Taufkirchen, Germany), diluted 1/8000 in Tris-buffered Saline, pH 8/0.05% Tween 20.

Thaumatin-like protein To32 detection was performed under reducing conditions. Samples were heated for 10 min at 95 °C before loading. Recombinant To32 was detected by using anti-FLAG M2 monoclonal antibody-peroxidase conjugate (A-8592, Sigma, Deisenhofen, Germany), diluted 1/1000 in Tris-buffered Saline, pH 8/0.05% Tween 20.

### Microscopy

Plant tissues were analysed using an Axiovert 200 (Zeiss, Germany). GFP fluorescence was visualised by using filter set 10 (excitation: 450-490 nm; emission: 515-565 nm, Zeiss, germany). Cell wall substances were stained by 5 µg/ml Fluorescent Brightener 28 (cellulose specific, Sigma, Deisenhofen, Germany) or 0.01% Aniline Blue (callose specific, Merck, Darmstadt, Germany) and regeneration of cell walls was analysed by using Filter set 2 (excitation: G 365 nm; emisson: LP 420nm, Zeiss, Germany).

### Results

### Viability

The viability of isolated moss protoplasts 48 hours after transformation was nearly 50%. Viability was calculated by counting the surviving protoplasts microscopically in a Fuchs-Rosenthal-chamber. Schaefer (Protocol 2001) mentioned a regeneration rate of 50 %.

### Transformation rate

Estimated by GFP expressing transiently transformed protoplasts 5 to 10 % of viable protoplasts were transformed. Transformation was performed with 60 µg DNA. The ratio of transformed protoplasts to surviving protoplasts was calculated by counting the surviving and GFP expressing protoplasts microscopically in a Fuchs-Rosenthal-chamber. Schaefer (Protocol 2001) mentioned a transformation rate of survived protoplasts of 5 to 25 %.

### Regeneration of protoplasts

Protoplasts could be cultivated in 3M medium or in W5 medium over 2 to 3 months. Cultivated in 3M or W5 medium, protoplasts were not able to regenerate cell walls or to divide. Nevertheless, transfer of 3 month old protoplasts into regeneration medium resulted in a slower but apparently normal regeneration. Regeneration of cell walls were verified by using specific dyes against callose (Aniline Blue) and cellulose (Fluorescent Brightener 28) and visualised by microscopy.

### Secretion

Deletion experiments were performed in order to analyse the proper cleavage of signal peptides and targeted secretion. The terminal amino acids of the signal peptides representing the cleavage sites were deleted.

### Example 1: human VEGF signal peptide

Human VEGF signal peptide-coding sequence was amplified by PCR using specific primers (MoB323 / MoB349; template: pRT101 P21 (Gorr 1999)) and introduced in frame to *vegf*121 into pRT101 C3 (Gorr 1999). The resulting plasmid pRT101TPVEGF C3 was used for transient transformation experiments.

Human VEGF signal peptide-coding sequence, non-comprising the three nucleotides (5'-CAG-3') coding for the C-terminal amino acid (Gln) was amplified by PCR using specific primers (MoB323/ MoB350) and introduced in frame to *vegf*121 into pRT101 C3. The resulting plasmid pRT101TPVEGF-1 C3 was used for transient transformation experiments as well.

60 µg DNA (purified by columns, Qiagen, Germany) of each construct was used for transformation. Three transformation experiments were done in parallel for each construct. The transformation samples were cultivated in 3M medium over 48 h in 48 well plates under dim light conditions (16 h light : 8 h dark). 24 h after transformation half of the medium was replaced by fresh medium. Sampling was performed 48 h after transformation by removing 200 µl. VEGF₁₂₁ targeted into the medium by the native signal peptide resulted in 8.47 ng/ml +/- 1.95 and was set to 100%. Deletion of the last amino acid of the signal peptide resulted in decreased secretion of 0.21 ng/ml +/- 0.06 respectively 2.48% (Tab. 1).

### Example 2: plant thaumatin like protein To32 signal peptide

Plant thaumatin like protein To32 signal peptide-coding sequence was amplified by PCR using specific primers (MoB254 / MoB250; template: pCRToN) and introduced in frame to *vegf*121 into pRT101 C3 (Gorr 1999). The resulting plasmid pRT101TPTo32 C3 was used for transient transformation experiments.
Plant thaumatin-like protein To32 signal peptide-coding sequence, not including the three nucleotides (CTG) coding for the C-terminal amino acid (Leu) was amplified by PCR using specific primers (MoB254 / MoB324) and introduced in frame to *vegf*121 into pRT101 C3. The resulting plasmid pRT101TPTo32-1 C3 was used for transient transformation experiments.

60 µg DNA (purified by columns, Qiagen, Germany) of each construct were used for transformation. Three transformation experiments were done in parallel for each construct. The transformation samples were cultivated in 3M medium over 48 h in 48 well plates under dim light conditions (16 h light : 8 h dark). 24 h after transformation half the medium was replaced by fresh medium. Sampling was performed 48 h after transformation by removing 200 µl. VEGF121 targeted into the medium by the native signal peptide yielded in 26.83 ng/ml +/- 6.25 and was set as 100%. Deletion of the last amino acid of the signal peptide resulted in decreased secretion of 2.18 ng/ml +/- 0.67 respectively 8.13%. (Tab. 1).

### Comparison of different signal peptides in transient expression assays

We investigated whether or not the use of signal peptides other than the native signal peptide results in improved secretion of recombinant proteins. The native signal peptide of VEGF was compared to thaumatin-like protein To32 signal peptide.

Human VEGF signal peptide-coding sequence was amplified by PCR using specific primers (MoB / MoB; template: pRT101 P21 (Gorr 1999)) and introduced in frame to *vegf*121 into pRT101 C3 (Gorr 1999). The resulting plasmid pRT101TPVEGF C3 was used for transient transformation experiments. Plant thaumatin like protein To32 signal peptide-coding sequence was amplified by PCR using specific primers (MoB / MoB; template: pCRToN) and introduced in frame to *vegf*121 into pRT101 C3 (Gorr 1999). The resulting plasmid pRT101TPTo32 C3 was used for transient transformation experiments.
60 µg DNA (purified by columns, Qiagen, Germany) of each construct were used for transformation. Three transformation experiments were done in parallel for each construct. The transformation samples were cultivated in 3M medium over 48 h in 48 well plates under dim light conditions (16 h light : 8 h dark). 24 h after transformation half the medium was replaced by fresh medium. Sampling was performed 48 h after transformation by removing 200 µl. VEGF121 targeted by plant thaumatin like protein To32 signal peptide into the medium resulted in three fold higher yields (123.3 ng/ml +/- 11.1) compared to targeting by the VEGF signal peptide (41.7 ng/ml +/- 6.9).
Secretion of recombinant protein was improved by using the non-native signal peptide (Tab.2).

### Time course experiments using different media

Human VEGF signal peptide-coding sequence was amplified by PCR using specific primers (MoB / MoB; template: pRT101 P21 (Gorr 1999)) and introduced in frame to *vegf*121 into pRT101 C3 (Gorr 1999). The resulting plasmid pRT101TPVEGF C3 was used for transient transformation experiments.
60 µg DNA (purified by Qiagen Midiprep) of pRT101TPVEGF C3 were used for transformation. Three transformation experiments were done in parallel for each cultivation condition. The transformation samples were cultivated in 3M medium or W5 medium or MMS medium over 96 h in 48 well plates under dim light conditions (16 h light : 8 h dark). Sampling was performed by replacing half of the medium (200 µl) by fresh medium every 24 h. Secretion rates were calculated as followed:
protein yield after 24 h = secretion rate 0-24 h (24 h);
protein yield after 48 h - (protein yield after 24 h / 2) = secretion rate 24-48 h (48 h);
protein yield after 72 h - (protein yield after 48 h / 2) = secretion rate 48-72 h (72 h);
protein yield after 96 h - (protein yield after 72 h / 2) = secretion rate 72-96 h (96 h);
secretion rates 0-24 h + 24-48 h + 48-72 h + 72-96 h = secretion rate 0-96 h (0-96 h).

Time courses for secretion were slightly different in the tested media. Transient expression performed in W5 medium (24 h =6.25 ng/ml) and MMS (24 h =5.12 ng/ml) medium started earlier compared to secretion rate 0-24 h in 3 M medium (24 h =1.72 ng/ml). But, more or less the same secretion rates could be observed in 3M medium between 24 and 96 h (48 h = 5.85 ng/ml; 72 h = 4.48 ng/ml; 96 h =4.3 ng/ml). In contrast, decreased secretion rates were measured between 72 and 96 h in transient transformation samples performed in W5 medium (48 h = 7.05 ng/ml; 72 h = 5.05 ng/ml; 96 h =2.43 ng/ml) and MMS medium (48 h = 4.54 ng/ml; 72 h = 4.68 ng/ml; 96 h =0.86ng/ml) (Tab.3).
Calculation of secretion rates over the entire 96 h (0-96 h) resulted in highest yields for W5 medium (20.77 ng/ml), followed by 3M medium (16.35 ng/ml) and MMS medium (15.2 ng/ml) (Tab.3). Use of different media for transient transformation of protoplasts resulted in different time courses of secretion over 96 h. In contrast, total yields of excreted recombinant protein after 96 h were only slightly different.

### Highly concentrated protein yields

60 µg DNA of the construct (pRT101TPTo32 C3 purified by Qiagen Midiprep) were used for each transformation.
The transformation samples were cultivated in 3M medium over 72 h in 96 well plates under dim light conditions (16 h light : 8 h dark). Medium was replaced by fresh medium 24 h after transformation. Supernatants (150 µl / transformation sample) were collected 48 h after transformation, new 3M medium was added and 72 h after transformation supernatants were collected again. Incubation of protoplasts in such a small volume resulted in 130 ng/ml, (mean value: 2 pg / cell) of excreted recombinant VEGF₁₂₁ during 24 h.

### Dimerization of VEGF₁₂₁ in moss protoplasts

In mammalian cells, VEGF₁₂₁ is synthesized as a precursor protein and after removal of the signal peptide it is processed to a disulfide-linked homodimer of 34 - 36 kDa. Here, we used VEGF leader peptide and thaumatin like protein To32 leader peptide to evaluate properly disulfide-linked dimerization and secretion of VEGF₁₂₁ expressed in transiently transformed moss protoplasts. The excreted protein was analysed in Western blot assays under nonreducing conditions. 60 µg DNA of the constructs (pRT101TPVEGF C3 and pRT101TPTo32 C3) were used for each transformation.

The transformation samples were cultivated in 3M medium over 72 h in 96 well plates under dim light conditions (16 h light : 8 h dark). 24 h after transformation medium was replaced by fresh medium. Supernatants were collected 48 h after transformation, new 3M medium was added and 72 h after transformation supernatants were collected again.

Under non-reducing conditions the antibody detected one band of 35 kDa. This suggests that the monomers were properly dimerised and efficiently secreted. *E*. *coli* derived recombinant VEGF₁₂₁ (refolded to the native structure, R&D Systems, Germany) was used as control.

### Quantitative determination of biologically active recombinant VEGF₁₂₁ expressed by moss

Biological activity was determined by RELIDA technology (RELIATech, Braunschweig, Germany). The RELIDA kit is based on soluble VEGF-A receptors as capture molecules of active ligands. The receptor recognize only free, uncomplexed and biologically active forms of VEGF-A (including VEGF₁₂₁). Compared to ELISA results the same amounts of VEGF₁₂₁ were measured with RELIDA. Based on this result, excreted recombinant VEGF₁₂₁ seems to be completely biologically active.

### Thaumatin like protein To32

Thaumatin like protein To32 showed CD4 binding capacity. For analytical purposes microgram amounts were required. The above described transient transformation of moss protoplasts was used for the production of excreted recombinant To32.

Transient expression of thaumatin like protein To32 was performed in 3 M medium. Protoplasts were isolated from bioreactor cultures (pH 4.5). 60 µg DNA (pRT101To32) were used for each transformation. The transformation samples were cultivated in 3M medium over 96 h in 96 well plates under dim light conditions (16 h light : 8 h dark). Supernatants (150 µl) were collected 24 h, 48 h, 72 h and 96 h after transformation. Collected supernatants were always replaced by identical volumes of fresh medium. During the first 24 h no recombinant To32 was excreted into the medium. After the first 24 h high yields of recombinant To32 were excreted continiously into the medium (24- 48 h = 2.5 µg (mean value: 37.5 pg/cell); 48-72 h = 5 µg (mean value: 75 pg/cell); 72 -96 h = 3.75 µg (mean value: 56.2 pg/cell; mean value). *E.coli* derived To32 was used for quantification in Western blot analyses.

With the transient transformation in moss protoplasts excreted recombinant protein yields up to 5 µg/ml per 24 h were achieved for a thaumatin like protein (To32) having CD4 binding capacity.

### Comparison to other systems

Fiebich et al. (1993) described recombinant expression and secretion of VEGF in baculovirus infected insect cells. They achieved levels of 3-5 µg VEGF / ml per 2-4 x 10⁶ cells (mean value per cell = 1.33 pg). In transient transformed moss protoplasts secretion levels of 130 ng VEGF / ml per 6.6 x 10⁴ cells (mean value per cell = 1.95 pg) were achieved.

Transient transformation of moss protoplasts with the coding sequence of thaumatin like protein To32 resulted in 5 µg /ml per 6.6 x 10⁴ cells (mean value per cell = 75 pg) during 24 h.

### Example 3: Transient Antibody production in Physcomitrella patens protoplasts

### Expression vectors

Expression vectors are generated by blunt cloning *Pfu*-proof-reading-PCR products of heavy (HC; 1410 bp) or light (LC; 711 bp) chain, respectively, in the SmaI restriction site of pRT101 (Töpfer et al. (1987), NAR, 15, p5890). As template serves the human kappa LC homologoue (GENBANK accession number BC005332, contained in IMAGE clone 3934658; sequence see below) and, in case of the HC, a synthetic DNA construct (sequence see below), which is a fusion of a human IgG HC precursor (GENBANK accession number AF135164) and the human IgG4 HC constant region (GENBANK accession number K01316). PCR conditions: 2 min 94°C; 30 cycles: 20s 94°C / 10s 50°C / 1 min/kb 72°C; extension: 10 min, using primers with 22 nucleotides in length, starting with the corresponding start or stop codon, respectively. In such expression constructs are located the CaMV 35S promoter, the HC or LC coding sequences including their corresponding endogenous signalpeptides (sorting signals for secretion), and as transcriptional terminator the CaMV 35S termination signal is used.

GENBANK accession number BC005332 (bp 21-731):

5'-
ATGGACATGAGAGTCCTCGCTCAGCTCCTGGGGCTCCTGCTGCTCTGTTTCCCAGGTGCCAGATGTGAC
ATCCAGATGACCCAGTCTCCATCCTCACTGTCTGCATCTGTCGGAGACACAGTCACCATCACTTGTCGG

GCGAGTCAGGACATTAGCAATTATTTAGCCTGGTTTCAGCAGAAACCAGGGAAAGCCCCTAAGTCCCTG
ATCTATGGTGCATCCAGTTTGCAAAGTGGGGTCCAATCAAAGTTCAGCGGCAGTGGATCTGGGACAGAT
TTCACTCTCACCATCAGCAGCCTGCAGCCTGAAGATTTTGCAACTTATTACTGCCAACAGTATAAAAGT
TATCCTGTCACTTTTGGCCAGGGGACCAAGCTGGAGATCAAACGAACTGTGGCTGCACCATCTGTCTTC
ATCTTCCCGCCATCTGATGAGCAGTTGAAATCTGGAACTGCCTCTGTTGTGTGCCTGCTGAATAACTTC
TATCCCAGAGAGGCCAAAGTACAGTGGAAGGTGGATAACGCCCTCCAATCGGGTAACTCCCAGGAGAGT
GTCACAGAGCAGGACAGCAAGGACAGCACCTACAGCCTCAGCAGCACCCTGACGCTGAGCAAAGCAGAC
TACGAGAAACACAAAGTCTACGCCTGCGAAGTCACCCATCAGGGCCTGAGCTCGCCCGTCACAAAGAGC
TTCAACAGGGGAGAGTGTTAG -3'

Synthetic HC construct:
bp 1-437: GENBANK accession number AF135164
bp 438-1410: GENBANK accession number K01316 (introns removed)

5'-
ATGGAGTTTGGGCTGAGCTGGCTTTTCCTCGTTGCTCTTTTAAGAGGTGTCCAGTGTCAGGTGCAGCTG
GTGGAGTCTGGGGGAGGCGTGGTCCAGCCTGGGAGGTCCCTGAGACTCTCCTGTGCAGCGTCTGGATTC
ACCTTCAGTAGCTATGGCATGCACTGGGTCCGCCAGTCTCCAGGCAAGGGGCTGGAGTGGGTGGCACTT
ATATGGTATGATGGAAGTAATAAATACTATGCAGACTCCGTGAAGGGCCGACTCACCATCTCCAGAGAC
AATTCCAAGAACACACTGTATCTGGAAATGAACAGCCTGAGAACCGAGGACACGGCCACATATTACTGT
GCGAAAGATCTCATGGCTCGGGGAGTCACCCCTGATGCTTTTGATATCTGGGGCCAAGGGACAAAGGTC
ACCGTCTCTTCAGCCTCCACCAAGGGCCCATCCGTCTTCCCCCTGGCGCCCTGCTCCAGGAGCACCTCC
GAGAGCACAGCCGCCCTGGGCTGCCTGGTCAAGGACTACTTCCCCGAACCGGTGACGGTGTCGTGGAAC
TCAGGCGCCCTGACCAGCGGCGTGCACACCTTCCCGGCTGTCCTACAGTCCTCAGGACTCTACTCCCTC
AGCAGCGTGGTGACCGTGCCCTCCAGCAGCTTGGGCACGAAGACCTACACCTGCAACGTAGATCACAAG
CCCAGCAACACCAAGGTGGACAAGAGAGTTGAGTCCAAATATGGTCCCCCATGCCCATCATGCCCAGCA
CCTGAGTTCCTGGGGGGACCATCAGTCTTCCTGTTCCCCCCAAAACCCAAGGACACTCTCATGATCTCC
CGGACCCCTGAGGTCACGTGCGTGGTGGTGGACGTGAGCCAGGAAGACCCCGAGGTCCAGTTCAACTGG
TACGTGGATGGCGTGGAGGTGCATAATGCCAAGACAAAGCCGCGGGAGGAGCAGTTCAACAGCACGTAC
CGTGTGGTCAGCGTCCTCACCGTCCTGCACCAGGACTGGCTGAACGGCAAGGAGTACAAGTGCAAGGTC
TCCAACAAAGGCCTCCCGTCCTCCATCGAGAAAACCATCTCCAAAGCCAAAGGGCAGCCCCGAGAGCCA
CAGGTGTACACCCTGCCCCCATCCCAGGAGGAGATGACCAAGAACCAGGTCAGCCTGACCTGCCTGGTC
AAAGGCTTCTACCCCAGCGACATCGCCGTGGAGTGGGAGAGCAATGGGCAGCCGGAGAACAACTACAAG

ACCACGCCTCCCGTGCTGGACTCCGACGGCTCCTTCTTCCTCTACAGCAGGCTAACCGTGGACAAGAGC
AGGTGGCAGGAGGGGAATGTCTTCTCATGCTCCGTGATGCATGAGGCTCTGCACAACCACTACACACAG
AAGAGCCTCTCCCTGTCTCTGGGTAAATGA -3'

### Transient Transformation

The protocol is the same as describe above.
In each transformation expression vectors for both chains, HC and LC, are co-transformed using 20 microgram of uncut supercoiled plasmid DNA per construct.
Samples are taken after 48 hours.

### Verification of product

### Detection of light and heavy chain assemblies

Coat: anti-human Kappa LC (Sigma, cat# K4377; 1/300 in coating buffer (s. below).

Conjugate: anti-human IgG4 peroxidase conjugate (Sigma, cat# A2290; 1:1000 in PBS (s. below) + 0,1% BSA).

Standard: human IgG4 Kappa (40ng/ml 20mM TBS (pH8,0) Sigma, cat# 14639), eight times serially diluted (in PBS + 0,1% BSA) 1:2 for the assay (up to 256 fold dilution).

Protocol: Coat 96-well ELISA plate with 50µl/well coating solution. Leave over night at 4°C. Solution 'flicked' off and washed three times at 100µl/well with PBS-T (s. below). Plate then blocked using 200µl/well PBS + 1%BSA. Leave 90 minutes at 37°C. Blocking solution 'flicked' off, washed three times at 100µl/well with PBS-T and 50µl/well standards / samples applied. Leave 90 minutes at 37°C. Plate washed three times at 100µl/well with PBS-T. Conjugate applied at 100µl/well. Leave 40 minutes at room temperature. Plate washed three times at 100µl/well with PBS-T. Ad 100 µl/well TMB (Trimethylbenzidin; Dunn Labortechnik; cat# TMBE-500), stop reaction after 10 minutes by adding 50µl/well 2M H2SO4. Measure absorption at 450 nm (reference wavelength: 610 nm; ELISA reader: Labsystems Multiskan RC).

coating buffer: titration of 70 ml 0,1M Na₂CO₃ (10,6 g/l, water free) mit 175 ml 0,2M NaHCO₃ (16,8 g/l, water free).

10xPBS: 20ml 1M KH₂PO₄ /80ml 1M K₂HPO₄ / 87,66 g NaCl ad 1000 ml PBS-T: PBS / 0,05 % (v/v) Tween 20

### Results

### Detection of light and heavy chain assemblies

Light and heavy chain assemblies are detected in several independent experiments.

### References

De Loose M, Tire GG, Gielen J, Villarroel R, Genetello C, Van Montagu M, Depicker A and Inze D (1991) The extensin signal peptide allows secretion of a heterologous protein from protoplasts. Gene 99, 95-100.
Denecke J, Botterman J and Deblaere R (1990) Protein secretion in plant cells can occur via default pathway. The Plant Cell 2, 51-59.
De Wilde C, De Rycke R, Beeckman T, De Neve M, Van Montagu M, Engler G and Depicker A (1998) Accumulation pattern of IgG antibodies and Fab fragments in transgenic Arabidopsis thaliana plants. Plant Cell Physiol 39, 639-646.
Dirnberger D, Steinkellner H, Abdennebi L, Remy J-J and van de Wiel D (2001) Secretion of biologically active glycoforms of bovine follicle stimulating hormone in plants. Eur J Biochem 268, 4570-4579.
Edman P and Begg G (1967) A protein sequenator. Eur J Biochem 1, 80-91.
Engel PP (1968) The induction of biochemical and morphological mutants in the moss Physcomitrella patens. Am J Bot 55, 438-446.
Fiebich BL, Jäger B, Schöllmann C, Weindel K, Wilting J, Kochs G, Marme D, Hug H and Weich HA (1993) Synthesis and assembly of functionally active human vascular endothelial growth factor homodimers in insect cells. Eur J Biochem 211, 19-26
Fischer R, Vaquero-Martin C, Sack M, Drossard J, Emans, N and Commandeur U (1999) Towards molecular farming in the future: transient protein expression in plants. Biotechnol Appl Biochem 30, 113-116
Fischer R and Emans N (2000) Molecular farming of pharmaceutical proteins. Transgenic Research 9, 279-299.
Gorr G (1999) Biotechnologische Nutzung von Physcomitrella patens (Hedw.) B.S.G.. Dissertation, Universität Hamburg.
Grimsley NH, Ashton NW and Cove DJ (1977) The production of somatic hybrids by protoplast fusion in the moss, Physcomitrella patens. Mol Gen Genet 154, 97-100.
Hohe A, Reski R (2002) Optimisation of a bioreactor culture of the moss Physcomitrella patens for mass production of protoplasts. Plant Sci 163, 69-74.
Hoube-Herin N, Pethe C, d'Alayer J, Laloue M (1999) Cytokinin Oxidase from Zea Mays: Purification, cDNA cloning and expression in moss Protoplasts. The Plant Journal 17(6), 615 - 626.
Kiessling J, Kruse S, Rensing SA, Harter K, Decker EL and Reski R (2000) Visualization of a Cytoskeleton-like FtsZ Network in Chloroplasts. J Cell Biol 151, 945-950.
Magnuson NS, Linzmaier PM, Gao J-W, Reeves R, An G and Lee JM (1996) Enhanced recovery of a secreted mammalian protein from suspension culture of genetically modified tobacco cells. Prot Expr Pur 7, 220-228.
Reski R, Abel WO (1985) Induction of budding on chloronemata and caulonemata of the moss, Physcomitrella patens, using isopentenyladenine. Planta 165, 354-358.
Reski R, Faust M, Wang X-H, Wehe M, Abel WO (1994) Genome analysis of the moss Physcomitrella patens (Hedw.) B.S.G.. Mol Gen Genet 244, 352-359.
Rother S, Hadeler B, Orsini JM, Abel WO and Reski R (1994) Fate of a mutant macrochloroplast in somatic hybrids. J Plant Physiol 143, 72-77.
Reutter K and Reski R (1996) Production of a heterologous protein in bioreactor cultures of fully differentiated moss plants. Plant Tissue Culture and Biotechnology 2, 142-147.
Schaefer D, Zryd J-P, Knight CD and Cove DJ (1991) Stable transformation of the moss Physcomitrella patens. Mol Gen Genet 226, 418-424.
Schaefer DG (2001) Principles and protocols for the moss Physcomitrella patens. http://www.unil.ch/lpc/docs/PPprotocols2001.pdf
Sheen J (2001) Signal transduction in maize and arabidopsis mesophyll protoplasts. Plant Physiol 127, 1466-1475.
Töpfer R, Matzeit V, Gronenborn B, Schell J and Steinbiss H-H (1987) A set of plant expression vectors for transcriptional and translational fusions. NAR 15, 5890.
Vaquero C, Sack M, Chandler J, Drossard J, Schuster F, Monecke M, Schillberg S and Fischer R (1999) Transient expression of a tumor-specific single-chain fragment and a chimeric antibody in tobacco leaves. Proc Natl Acad Sci USA 96, 11128-11133.
Zeidler M, Hartmann E and Hughes J (1999) Transgene expression in the moss Ceratodon purpureus. J Plant Physiol 154, 641-650.

**Table 1: Transient expression and secretion of VEGF₁₂₁ in moss protoplasts using different leader peptide sequences and deletions thereof.**

| construct | mean value rh VEGF | percent |
|---|---|---|
| pRT101TPVEGF C3 | 8.47 ng/ml +/- 1.95 | 100 |
| pRT101TPVEGF-1 C3 | 0.21 ng/ml +/- 0.06 | 2.48 |
| construct | mean value rh VEGF | percent |
| pRT101TPTo32 C3 | 26.83 ng/ml +/- 6.25 | 100 |
| pRT101TPTo32-1 C3 | 2.18 ng/ml +/- 0.67 | 8.13 |

Construction of vectors is described in Methods and Material. Amounts of secreted VEGF₁₂₁ were measured by ELISA. Mean values and standard deviation were calculated from three transformation experiments done in parallel.

**Table 2: Transient expression and secretion of VEGF₁₂₁ in moss protoplasts using different leader peptide sequences.**

| construct | mean value rh VEGF | percent |
|---|---|---|
| PRT101TPVEGF C3 | 41.7 ng/ml +/- 6.9 | 100 |
| PRT101TPTo32 C3 | 123.3 ng/ml +/- 11.1 | 295 |

Construction of vectors is described in Methods and Materials. Amounts of secreted VEGF₁₂₁ were measured by ELISA. Mean values and standard deviation were calculated from three transformation experiments done in parallel.

**Table 3: Secretion rates (per 24 h) of VEGF₁₂₁ in transiently transformed moss protoplasts cultured in different media.**

| medium | 24 h | 48 h | 72 h | 96 h | total yield |
|---|---|---|---|---|---|
| 3M | 1.72 ng/ml | 5.85 ng/ml | 4.48 ng/ml | 4.3 ng/ml | 16.35 ng/ml |
| W5 | 6.25 ng/ml | 7.05 ng/ml | 5.05 ng/ml | 2.43 ng/ml | 20.77 ng/ml |
| MMS | 5.12 ng/ml | 4.54 ng/ml | 4.68 ng/ml | 0.86 ng/ml | 15.20 ng/ml |

Secretion rates are calculated as described in Methods and Materials. Mean values were calculated from three transformation experiments done in parallel. For transformation the construct pRT101TPVEGF was used.

## Claims

1. A method for the transient production of (an) extracellular non-plant protein(s) in a moss protoplast, which method comprises the steps of transiently transforming the protoplast with a nucleic acid construct comprising (a) heterologous nucleotide sequence(s) coding for said non-plant protein(s) operably linked to a promoter, and cultivating the transformed protoplast in order to produce said non-plant protein(s), wherein said cultivation takes place for at least 48 hours in a culture medium selected from the group consisting of W5, 3M and MMS.

2. A method according to claim 1, further comprising the step of obtaining said non-plant protein(s) from the culture medium.

3. A method as claimed in claim 1 or 2, wherein the protoplast is from *Physcomitrella patens.*

4. A method as claimed in any of claims 1 to 3, wherein the culture medium volume is in the range of from 50µl - 1000µl.

5. A method as claimed in any of claims 1 to 4, wherein the culture medium volume is in the range of from 100µl - 600µl.

6. A method as claimed in any of claims 1 to 5, wherein the culture medium volume is in the range of from 100µl - 300µl.

7. A method as claimed in any of claims 1 to 6, wherein the culture medium volume is 200µl +/- 50µl.

8. A method as claimed in any one of the preceding claims, wherein the heterologous nucleic acid comprises two or more heterologous nucleotide sequences.

9. A method as claimed in claim 8, wherein the nucleic acids are present in a single vector construct.

10. A method as claimed in any one of the preceding claims, wherein the promoter is an inducible promoter.

11. A method as claimed in any one of the preceding claims, wherein the heterologous nucleotide sequence encodes a protein or polypeptide which is an animal protein.

12. A method as claimed in any one of the preceding claims, wherein the heterologous nucleotide sequence encodes a protein or polypeptide which is a mammalian protein.

13. A method as claimed in claim 11 or 12, wherein the heterologous nucleotide sequence encodes a protein selected from the group consisting of a heterodimer, a fusion antibody, an immunoglobulin, and a single chain antibody.

14. A method as claimed in any one of claims 1 to 10, wherein the heterologous nucleotide sequence encodes a protein or polypeptide which is a fungal protein.

15. A method as claimed in any one of claims 1 to 10, wherein the heterologous nucleotide sequence encodes a protein or polypeptide which is a bacterial protein.

## Patentansprüche

1. Verfahren zur transienten Herstellung eines extrazellulären, nicht-pflanzlichen Proteins in einem Moosprotoplasten, wobei das Verfahren die Schritte der transienten Transformation des Protoplasten mit einem Nukleinsäurekonstrukt, welches eine für das nicht-pflanzliche Protein kodierende und operabel mit einem Promotor verknüpfte heterologe Nukleotidsequenz umfasst, und der Kultivierung des transformierten Protoplasten zur Herstellung des nicht-pflanzlichen Proteins umfasst, wobei die Kultivierung mindestens 48 Stunden lang in einem aus der Gruppe bestehend aus W5, 3M und MMS ausgewählten Medium erfolgt.

2. Verfahren nach Anspruch 1, ferner umfassend den Schritt der Gewinnung des nicht-pflanzlichen Proteins aus dem Kulturmedium.

3. Verfahren nach Anspruch 1 oder 2, wobei der Protoplast von *Physcomitrella patens* ist.

4. Verfahren nach einem der Ansprüche 1 bis 3, wobei das Volumen des Kulturmediums im Bereich von 50 µl bis 1000 µl liegt.

5. Verfahren nach einem der Ansprüche 1 bis 4, wobei das Volumen des Kulturmediums im Bereich von 100 µl bis 600 µl liegt.

6. Verfahren nach einem der Ansprüche 1 bis 5, wobei das Volumen des Kulturmediums im Bereich von 100 µl bis 300 µl liegt.

7. Verfahren nach einem der Ansprüche 1 bis 6, wobei das Volumen des Kulturmediums 200 µl +/- 50 µl beträgt.

8. Verfahren nach einem der vorhergehenden Ansprüche, wobei die heterologe Nukleinsäure zwei oder mehr heterologe Nukleotidsequenzen umfasst.

9. Verfahren nach Anspruch 8, wobei die Nukleinsäuren in einem einzigen Vektorkonstrukt vorliegen.

10. Verfahren nach einem der vorhergehenden Ansprüche, wobei der Promotor ein induzierbarer Promotor ist.

11. Verfahren nach einem der vorhergehenden Ansprüche, wobei die heterologe Nukleotidsequenz für ein tierisches Protein oder Polypeptid kodiert.

12. Verfahren nach einem der vorhergehenden Ansprüche, wobei die heterologe Nukleotidsequenz für ein Säuger-Protein oder -Polypeptid kodiert.

13. Verfahren nach Anspruch 11 oder 12, wobei die heterologe Nukleotidsequenz für ein Protein kodiert, welches aus der Gruppe bestehend aus einem Heterodimer, einem Fusionsantikörper, einem Immunglobulin, und einem Single-Chain-Antikörper ausgewählt ist.

14. Verfahren nach einem der Ansprüche 1 bis 10, wobei die heterologe Nukleotidsequenz für ein fungales Protein oder Polypeptid kodiert.

15. Verfahren nach einem der Ansprüche 1 bis 10, wobei die heterologe Nukleotidsequenz für ein bakterielles Protein oder Polypeptid kodiert.

## Revendications

1. Une méthode pour la production transitoire d'une (de) protéine(s) extracellulaire(s) non-végétale(s) dans un protoplaste de mousse, laquelle méthode comprend les étapes de transformation transitoire le protoplaste avec une construction d'acide nucléique contenant une (des) séquence(s) nucléotidique(s) hétérologue(s) codant pour la(les) dite(s) protéine(s) non-végétale(s), liée(s) de manière opérationnelle à un promoteur, et de culture du protoplaste transformé de manière à produire la (les) dite(s) protéine(s) non-végétale(s), laquelle dite culture est maintenue pendant au moins 48 heures dans un milieu de culture sélectionné dans un groupe constitué de W5, 3M et MMS.

2. La méthode selon la revendication 1, comprenant de plus l'étape de récolte de la (des) dite(s) protéine(s) non-végétale(s) du milieu de culture.

3. La méthode selon les revendications 1 ou 2, dans laquelle le protoplaste provient de *Physcomitrella patens.*

4. La méthode selon une quelconque des revendications 1 à 3, dans laquelle le volume du milieu de culture est compris dans l'intervalle de 50 µl - 1000 µl.

5. La méthode selon une quelconque des revendications 1 à 4, dans laquelle le volume du milieu de culture est compris dans l'intervalle de 100 µl - 600 µl.

6. La méthode selon une quelconque des revendications 1 à 5, dans laquelle le volume du milieu de culture est compris dans l'intervalle de 100 µl - 300 µl.

7. La méthode selon une quelconque des revendications 1 à 6, dans laquelle le volume du milieu de culture est 200 µl +/-50 µl.

8. La méthode selon une quelconque des revendications précédentes, dans laquelle l'acide nucléique hétérologue comprend deux ou plus séquences nucléotidiques hétérologues.

9. La méthode selon la revendication 8, dans laquelle les acides nucléiques sont présents dans une construction de vecteur unique.

10. La méthode selon une quelconque des revendications précédentes, dans laquelle le promoteur est un promoteur inductible.

11. La méthode selon une quelconque des revendications précédentes, dans laquelle la séquence nucléotidique hétérologue code pour une protéine ou un polypeptide qui est une protéine animale.

12. La méthode selon une quelconque des revendications précédentes, dans laquelle la séquence nucléotidique hétérologue code pour une protéine ou un polypeptide qui est une protéine de mammifère.

13. La méthode selon une des revendications 11 ou 12, dans laquelle la séquence nucléotidique hétérologue code pour une protéine sélectionnée dans un groupe constitué d'un hétéro-dimère, un anticorps fusionné, une immunoglobuline, et un anticorps à chaîne unique.

14. La méthode selon une quelconque des revendications 1 à 10, dans laquelle la séquence nucléotidique hétérologue code pour une protéine ou un polypeptide qui est une protéine de champignon.

15. La méthode selon une quelconque des revendications 1 à 10, dans laquelle la séquence nucléotidique hétérologue code pour une protéine ou un polypeptide qui est une protéine de bactérie.
